# EUROPÄISCHE PATENTANMELDUNG

(11) **EP 1 712 689 A2**
(43) Veröffentlichungstag der Anmeldung: **18.10.2006**
(21) Anmeldenummer: 06113855.8
(22) Anmeldetag: 30.01.2004
(51) Int. Cl.: E03D 9/05, F24F 3/16

(54) **Deodosierungsvorrichtung für Toiletten**

(30) Priorität: 03.02.2003 DE 20301640 U
(62) Teilanmeldung aus: 04002036.4
(71) Anmelder: VILLEROY & BOCH AG, 66688 Mettlach (DE)
(72) Erfinder: CZAPLA, Christian, 66793, Saarwellingen (DE); BECKER, Ralf, 66663, Merzing (DE)
(74) Vertreter: Gritschneder, Martin

(57) **Zusammenfassung**

Zum Vermeiden von lästigen Gerüchen auf Toiletten weist die Toilettenausstattung eine Deodorisierungseinrichtung (10) auf, die über ein Ansaugrohr (22) Luft aus dem Inneren des WC-Beckens (34) absaugt. Die Deodorisierungseinrichtung (10) weist einen Lüfter (18) zum Absaugen von Luft aus dem Innenbereich des WC-Beckens (34) auf, sowie eine Ionisationseinrichtung (24) zum Erzeugen von Luftionen. Die abgesaugte Luft wird mit den erzeugten Luftionen in Kontakt gebracht. Ein Sensor (16) untersucht die abgesaugte Luft auf oxidierbare Geruchsmoleküle und erzeugt ein Signal, das die Konzentration oxidierbarer Geruchsmoleküle in der abgesaugten Luft wiedergibt. Die Ionisationseinrichtung (24) wird in Abhängigkeit von dem Signal des Sensors (16) gesteuert.

## Beschreibung

Die Erfindung betrifft eine Toilettenausstattung zum Vermeiden von lästigen Gerüchen auf Toiletten. Die Toilettenausstattung weist ein WC-Becken, eine Spüleinrichtung, die über ein Spülrohr das WC-Becken spült, und eine Deodorisierungseinrichtung auf, die mittels eines Lüfters über ein Ansaugrohr Luft aus dem Inneren des WC-Beckens absaugt. Die Deodorisierungseinrichtung enthält eine Ionisationseinrichtung zum Erzeugen von Luftionen. Die abgesaugte Luft wird mit den erzeugten Luftionen in Kontakt gebracht, wodurch die Geruchsmoleküle oxidiert und damit zerstört werden.

Aus US-A-2 001 592 ist eine Vorrichtung bekannt, bei der aus dem Inneren des WC-Beckens die Luft mittels des Lüfters abgesaugt wird und durch die Ionisationseinrichtung geleitet wird, um Geruchsmoleküle zu oxidieren. Der Lüftermotor und die Ionisationseinrichtung werden durch einen Schalter eingeschaltet, der durch das Herunterklappen des WC-Sitzbrettes betätigt wird. Der Lüfter und die Ionisationseinrichtung sind in einem Gehäuse untergebracht, das seitlich neben der Schwenkachse des WC-Sitzbrettes angebracht ist.

Aus US-A-6 163 893 ist es bei einer weiteren solchen Vorrichtung bekannt, die Luft über mehrere Öffnungen im WC-Sitzbrett anzusaugen und dann durch die Ozon erzeugende Einrichtung und ein Aktivkohlefilter zu schicken.

Aus JP-A-05168687 ist es bei einer solchen Vorrichtung bekannt, die Luft noch durch einen Katalysator zu leiten. Die Betriebsstunden des Katalysators werden aufsummiert, und die Arbeitsweise der Ionisationseinrichtung wird der abnehmenden Leistungsfähigkeit des Katalysators angepasst.

Aus EP-B1-0 331 192, Fig. 12, ist eine ähnliche Anordnung bekannt, wobei die Luft durch das Überlaufrohr des Spülkastens abgesaugt wird und aus dem Spülkasten durch die Ionisiereinrichtung und einen Katalysator gesaugt wird. Die Steuerung der Anlage erfolgt über einen Licht-Detektor am Sitzbrett.

Aus JP-A-03290538 ist eine solche Toiletten-Deodorierungsvorrichtung bekannt, bei der die Benutzung der Toilette mittels eines Sensors erkannt wird und daraufhin die Vorrichtung eingeschaltet wird. Nach Beendigung der Benutzung bleibt sie noch eine vorgegebene Zeitspanne eingeschaltet. Ähnliche Vorrichtungen sind auch aus JP-A-2001262663, JP-A-09195359, JP-A-07279213 und JP-A-07268928 bekannt.

Aus EP-A-0567775 ist es bekannt, Raumluft für Wohn- und Arbeitsräume mittels eines Klimazentralgerätes aufzubereiten. Vor der Behandlung wird die Raumluft mit einem Schadstoff-Sensor auf oxidierbare Schadstoffe analysiert. Die Schadstoffe werden mittels Ozon aus einem Oxidator oxidiert, wobei die Menge des im Oxidator erzeugten Ozons in Abhängigkeit von dem Signal des Schadstoff-Sensors gesteuert wird. Die Raumluft wird ferner in einer Filtereinrichtung behandelt, wobei noch vorhandenes Ozon in stabilen, molekularen Sauerstoff umgewandelt wird. Das in dem Ozonisator erzeugte Ozon wird zusätzlich von einem Ozon-Sensor gesteuert und geregelt, der nach der Filtereinrichtung angeordnet ist.

Der Erfindung liegt die Aufgabe zugrunde, eine Toilettenausstattung zu schaffen, mittels der einerseits lästige Gerüche sehr wirksam und zuverlässig unterdrückt werden können und anderseits die Produktion von überschüssigem Ozon vermieden wird.

Bei einer Toilettenausstattung der eingangs genannten Art wird diese Aufgabe dadurch gelöst, dass mittels eines Sensor die abgesaugte Luft auf oxidierbare Geruchsmoleküle untersucht wird und ein Signal erzeugt wird, das die Konzentration oxidierbarer Geruchsmoleküle in der abgesaugten Luft wiedergibt, wobei die Ionisationseinrichtung in Abhängigkeit von dem Signal des Sensors gesteuert wird.

Dadurch wird erreicht, dass die von der Ionisationseinrichtung produzierte Menge an Luftionen und Ozon gerade etwa so groß ist, wie es zur Oxidation der Geruchsmoleküle notwendig ist. Die Erzeugung einer wesentlichen Menge überschüssiger Luftionen und überschüssigen Ozons und der damit verbundene beißende Geruch werden vermieden.

Um die Benutzung der Toilette festzustellen und die Vorrichtung zu aktivieren, kann unter dem Sitzbrett ein Schalter angeordnet sein, durch den beim Niederdrücken des Sitzbrettes ein Stromkreis geschlossen wird. Es können aber auch bekannte Einrichtungen wie kapazitive Sensoren oder Infrarotsensoren verwendet werden, um die Gegenwart einer Person und damit die Benutzung der Toilette festzustellen.

In einer bevorzugten Ausgestaltung der Erfindung wird auch der Lüfter mittels des Sensorsignals gesteuert, z.B. in der Weise, dass der Lüfter mit einer ersten, relativ niedrigen Leistung arbeitet, wenn das Steuersignal keine oxidierbaren Geruchsmoleküle anzeigt, und dass er mit einer zweiten, höheren Leistung arbeitet, wenn das Steuersignal das Vorhandensein oxidierbarer Geruchsmoleküle in der abgesaugten Luft anzeigt.

Vorzugsweise arbeitet der Lüfter im Bereitschaftszustand, d.h., wenn der Sensor keine Geruchsmoleküle feststellt, etwa mit halber Leistung, während er dann, wenn der Sensor das Vorhandensein von Geruchsmolekülen feststellt, auf volle Leistung umgeschaltet wird. Gleichzeitig mit dem Hochfahren des Lüfters wird die Ionisierungseinrichtung aktiviert. Die Deodorisierungsvorrichtung fängt dadurch praktisch verzögerungsfrei zu arbeiten an. Ein weiterer Vorteil besteht darin, dass die Deodorisierungsvorrichtung nur aktiviert wird, wenn es tatsächlich notwendig ist.

Dadurch, dass der Sensor bei dieser Ausgestaltung der Erfindung ständig aktiv ist und der Lüfter im Bereitschaftszustand kontinuierlich mit niedriger Leistung läuft, spricht eine solche Deodorisierungsvorrichtung sehr rasch und automatisch an, wenn die Toilette benutzt wird. Zusätzliche Erkennungseinrichtungen oder Schalter am WC-Sitzbrett sind nicht notwendig.

Elektronische Steuerungen müssen von Zeit zu Zeit rückgesetzt werden, um Fehlfunktionen zu vermeiden. Ein solcher Reset findet zwangsläufig beim Einschalten des Gerätes statt. Da bei dieser Ausgestaltung der Erfindung das Deodorisierungsmodul ständig eingeschaltet ist, ist die Steuerung so ausgelegt, dass in bestimmten Zeitabständen, z.B. alle 24 Stunden, ein Reset durchgeführt wird.

Der Sensor kann in Richtung der von dem Lüfter erzeugten Luftströmung vor, hinter oder neben der Ionisierungseinrichtung angeordnet sein. Wichtig ist nur, dass der Sensor innerhalb des von dem Lüfter erzeugten Luftstroms angeordnet ist, bevor dieser Luftstrom wieder in den Raum austritt. Wenn der Sensor hinter der Ionisierungseinrichtung angeordnet ist, so ist bei der Festlegung der Steuerungskurve zu berücksichtigen, dass ein Teil der oxidierbaren Geruchsmoleküle bereits aufoxidiert ist, bevor sie den Sensor erreichen konnten. Die Abhängigkeit der Leistung der Ionisierungseinrichtung von dem Sensorsignal hat dann zumindest zum Teil den Charakter einer Regelung.

Der Sensor hat insgesamt eine dreifache Funktion:
Erstens wird die Ionisierungseinrichtung eingeschaltet, sobald der Sensor oxidierbare Geruchsmoleküle feststellt, zweitens steuert der Sensor in Abhängigkeit von der Konzentration der festgestellten Geruchsmoleküle die Leistung der Ionisierungseinrichtung und drittens wird die Leistung des Lüfters erhöht, sobald der Sensor oxidierbare Geruchsmoleküle feststellt.

Der Sensor ist vorzugsweise ein Halbleiter-Zinnoxid-Sensor, der oxidierbare Gasbestandteile in der Luft feststellt.

Derartige Sensoren sind allgemein bekannt und zum Beispiel von der Firma Fis Inc., 2-5-26, Hachizuka, Ikeda, Osaka, 563-0024 Japan, erhältlich. In einer besonders bevorzugten Ausführungsform der Erfindung wird ein H₂-Sensor verwendet. Wasserstoff ist zwar ein geruchloses Gas, kann jedoch als Leitgas verwendet werden, da es immer bei der menschlichen Verdauung gebildet wird, während die anderen Gasbestandteile z. B. Methan oder H₂S in Abhängigkeit von der aufgenommenen Nahrung im größeren oder kleineren Umfang oder überhaupt nicht gebildet werden. Gleichzeitig hat Wasserstoff den Vorteil, dass es nicht in Parfums und ähnlichen Kosmetika verwendet wird, so dass ein fälschliches Ansprechen des Sensors auf derartige Kosmetika verhindert wird. Ein H₂-Sensor hat damit den Vorteil, dass er einerseits sicher anspricht, wenn irgendwelche Geruchsmoleküle oder Luftbestandteile vorhanden sind, die von der menschlichen Verdauung herrühren, und andererseits ein fälschliches Ansprechen auf z. B. Kosmetika vermieden wird. Geeignete H₂-Sensoren sind ebenfalls von der Firma Fis Inc. erhältlich (siehe Products Review, Sensors and Systems Technology, revised June, 1998, Version 4.2 der Firma Vis Inc.), z. B. das Modell SB 19.

Die Deodorisierungsvorrichtung ist zweckmäßig als ein kompaktes Modul ausgebildet, das den Lüfter, den Sensor, die Ionisierungseinrichtung mit Ionisierungsröhre und Hochspannungseinheit, die Steuerung und die Stromversorgung für diese Komponenten enthält. Die Stromversorgung erfolgt dabei zweckmäßig über das Stromnetz mittels eines Netzteils mit einem Netztrafo. Der Lüfter saugt über ein Ansaugrohr Luft an und fördert sie zu dem Sensor und der Ionisierungseinrichtung.

Die Ionisierungsröhre kann ein Glas-Hohlzylinder mit einem Außendurchmesser von 20 mm, einer Zylinderhöhe von 50 mm und einer Wandstärke von 0,8 mm sein. Auf der Innen- und Außenseite des Glaszylinders sind flächige, gitterförmige Elektroden angebracht. Die Enden der zylinderförmigen Röhre können offen oder verschlossen sein.

Die Hochspannungseinheit der Ionisierungseinrichtung erzeugt eine Spannung von 1 bis 1,8 kV mit einer Frequenz von 8 bis 13 kHz, bei der vorausgehend beschriebenen Ionisierungsröhre vorzugsweise eine Wechselspannung von 1,5 kV bei einer Frequenz von 10 kHz. Die Wechselspannung wird in Form von Rechteckimpulsen mit einer Frequenz z.B. im Bereich von 50 Hz an die Elektroden der Ionisationsröhre angelegt. Die Steuerung der Ionisationsleistung, d.h. der Menge der erzeugten Luftionen und der Ozonmoleküle, wird dadurch gesteuert, dass die Dauer der Rechteckimpulse und deren Abstand verändert werden (Tastverhältnis). An der Ionisationsröhre liegt damit entweder keine Spannung an oder eine bestimmte fest eingestellte Hochspannung, z.B. 1,5 kV. Wenn Ionisation stattfindet, dann erfolgt sie dadurch mit der einmal gefundenen optimalen Spannung, bei der die Menge der erzeugten Luftionen und der Ozonmoleküle pro Zeiteinheit weitgehend konstant ist.

Der Lüfter kann einen Axiallüfter mit einer maximalen Leistung von etwa 9 bis 12 Liter pro Minute sein. Über das Ansaugrohr wird die Luft aus dem Inneren des WC-Beckens angesaugt und zu dem Deodorisierungsmodul geleitet. Das Ansaugrohr kann ein eigenes Rohr sein. Mit geeigneten Adaptern kann auch das Spülrohr oder das Überlaufrohr als Ansaugrohr fungieren, wie nachfolgend noch detaillierter ausgeführt wird. Die behandelte Luft wird über eine Auslassöffnung in dem Gehäuse des Deodorisierungsmoduls ausgeblasen, wobei diese Öffnung im Allgemeinen der Öffnung diametral gegenüberliegt, an der das Ansaugrohr in das Gehäuse mündet. Die behandelte Luft kann auch über ein Auslassrohr in den Raum abgeleitet werden. Dadurch wird die Zeitspanne verlängert, die zur Verfügung steht, um die oxidierbaren Geruchsmoleküle aufzuoxidieren. Die Geruchsmoleküle und die Luftionen und Ozonmoleküle sind während der Durchströmung des Auslassrohrs noch räumlich eingeschlossen, so dass sie miteinander reagieren können. Als ein solcher Reaktionsraum kann auch der Hohlraum auf der Rückseite eines WC-Beckens oder der Raum innerhalb eines Spülkastens dienen.

Die Toilettenausstattung weist ferner ein übliches WC-Becken und eine Spüleinrichtung mit einem Spülrohr auf. Bei der Spüleinrichtung kann es sich insbesondere um einen Spülkasten handeln, der über das Spülrohr mit dem WC-Becken verbunden ist.

In einer ersten, besonders einfachen Ausgestaltung der erfindungsgemäßen Toilettenausstattung ist das Deodorisierungsmodul (DOM) im hinteren Bereich des WC-Beckens angeordnet, bspw. unterhalb des Abflussrohrs oder Geruchsverschlusses, wo im allgemeinen ausreichend Platz zur Verfügung steht. Angesaugt wird die Luft aus dem Inneren des WC-Beckens über einen innerhalb des WC-Sitzes verlaufenden Kanal, der über eine Gelenkverbindung im Scharnier des WC-Sitzes mit dem Ansaugrohr des Deodorisierungsmoduls verbunden ist. In der Keramik des WC-Beckens kann eine zusätzliche, nach unten führende Öffnung im Bereich der Scharnierbefestigung vorgesehen sein. Statt über einen Kanal im WC-Sitz die Luft abzusaugen, besteht auch die Möglichkeit, eine eigene Öffnung neben dem Spülrohrauslass im oberen Bereich des WC-Beckens vorzusehen, über die die Luft abgesaugt und zum Deodorisierungsmodul geführt wird. An einem eventuell vorhandenen Spülkasten und an den Anschlüssen des Spülkastens und des Abflusses sind dann keine Veränderungen notwendig.

In einer zweiten Ausgestaltung der Erfindung ist das Deodorisierungsmodul im unteren Bereich des Spülkastens oder im hinteren Bereich des WC-Beckens neben oder unter dem WC-Abflussstutzen angeordnet und wird die Luft aus dem WC-Becken über den Spülrohrauslass abgesaugt und über eine Abzweigung aus dem Spülrohr zum Deodorisierungsmodul geleitet.

Bei dieser zweiten Ausgestaltung der Erfindung werden zweckmäßig Vorkehrungen getroffen, damit kein Wasser beim Spülen des WC über die Abzweigung zum Deodorisierungsmodul gelangt. Dazu kann zum Beispiel eine Verbindung von dem Spülrohr über ein U-Stück, das als Geruchsverschluss fungiert, zu dem WC-Abfluss vorhanden sein. Über dem Niveau des WC-Abflusses ist dann von dieser Verbindung zwischen Spülrohr und WC-Abfluss die Abzweigung zum Deodorisierungsmodul vorgesehen, die zweckmäßig zunächst etwas ansteigt.

Um das Deodorisierungsmodul vor Wasser zu schützen, kann auch ein Magnetventil oder ein rein mechanisches Ventil, zum Beispiel ein Kugelventil, ein Kippventil oder ein Torsionsventil, vorgesehen sein. Schließlich kann der Anfang der Abzweigung aus dem Spülrohr auch als Venturi-Ventil ausgebildet sein, d.h., das Einlassende der Abzweigung endet nicht stumpf an dem Spülrohr, sondern verläuft innerhalb des Spülrohrs ein kurzes Stück in Strömungsrichtung, so dass die Öffnung der Abzweigung von der Strömung des Spülwassers abgewandt ist.

Bei einer dritten Ausgestaltung der Erfindung verläuft das Ansaugrohr vom Rand des WC-Beckens innerhalb des waagerechten Teils des Spülrohrs zurück, folgt dem Spülrohr ein kurzes Stück innerhalb des senkrechten Teils, so dass kein Wasser über das Ansaugrohr zum Deodorisierungsmodul gelangen kann, und tritt dann seitlich aus dem Spülrohr aus und führt zum Deodorisierungsmodul. Der Austritt aus dem Spülrohr kann auch in den waagerechten Teil zurück verlegt sein, wozu das Ansaugrohr nach dem kurzen senkrechten Anstieg in einer U-Krümmung wieder zurück zum waagerechten Teil verläuft und dort seitlich austritt.

Bei einer vierten Ausgestaltung der Erfindung befindet sich das Deodorisierungsmodul im Spülkasten über dessen Flutungslinie. Als Ansaugrohr fungieren das Spülrohr und das Überlaufrohr, das unterhalb des Spülventils von dem Spülrohr abzweigt. Mittels eines Adapters wird das obere Ende des Überlaufrohrs in einem U-förmigen Kanal nach unten umgeleitet. Das Deodorisierungsmodul wird auf den Adapter aufgesetzt, wobei die Ansaugöffnung des Deodorisierungsmoduls in den U-förmigen Kanal mündet. Bei gefülltem Spülkasten wird Luft über das Überlaufrohr und das Spülrohr nur aus dem Inneren des WC-Beckens angesaugt, da das nach unten zeigende Ende des U-förmigen Kanals von dem im Spülkasten stehenden Wasser verschlossen ist.

Bei einer fünften Ausgestaltung der Erfindung ist das Ansaugrohr in dem Überlaufrohr und dem Spülrohr des Spülkastens nach unten geführt, wobei sich die Einlassöffnung des Ansaugrohrs im Spülkasten über dessen Flutungslinie befindet. Das Ansaugrohr tritt zwischen Spülkasten und WC-Becken aus dem Spülrohr aus und verläuft zum Deodorisierungsmodul, das sich in dem Hohlraum auf der Rückseite des WC-Beckens befindet.

Das erfindungsgemäße Deodorisierungsmodul ist in jeder der Ausgestaltungen dasselbe. Es ist sowohl für WC-Becken mit Wandmontage als auch für Kombi-WCs geeignet. Bei der Wandmontage wird es entweder mittels einer Manschette vom Abflussrohr abgehängt, wobei das Ansaugrohr dann an der Oberseite des aus der Wand herausgeführten Teils des Spülrohrs angesetzt ist und um das Abflussrohr herum zu dem Deodorisierungsmodul verläuft. Mittels elastischer Klemmelemente kann es auch in den Hohlraum auf der Rückseite des WC-Beckens eingesetzt werden. Die Klemmelemente können einfache Kunststoffstücke sein, die in ihrer Stärke an die Abmessungen des Hohlraums angepasst werden. Sie können auch seitlich von dem Deodorisierungsmodul abstehende Schrauben sein, deren Kopf mit einer Kunststoffauflage gegen die Innenseite des Hohlraums drückt. Durch Eindrehen bzw. Herausdrehen können die Schrauben an die Abmessungen des Hohlraums angepasst werden.

Bei einigen der erwähnten Ausgestaltungen der Erfindung verläuft das Ansaugrohr innerhalb des Spülrohrs oder Überlaufrohrs. Deren Querschnitt muss ggf. vergrößert werden, damit das Spülwasser genügend schnell in das WC-Becken einläuft. Die lichte Weite des Ansaugrohrs wird jeweils in Abhängigkeit von seiner Länge so gewählt, dass die gewünschte Ansaugleistung von etwa 9 bis 12 Liter pro Minute erreicht wird.

Es kann sich bisweilen das Problem ergeben, dass die Luft nach dem Durchgang durch die Deodorisierungsvorrichtung noch einen geringen Anteil an Ozon enthält. In diesen Fällen kann es vorteilhaft sein, der Deodorisierungsvorrichtung am Auslassende noch ein Aktivkohlefilter nachzuschalten. Da der größte Anteil der oxidierbaren Gase und des Ozons bereits vor dem Aktivkohlefilter miteinander reagieren, wird das Aktivkohlefilter wenig belastet, so dass es eine sehr hohe Standzeit hat. In bekannter Weise kann das Aktivkohlefilter z.B. durch Erhitzen regeneriert werden.

Die Deodorisierungsvorrichtung kann in bestehende WCs eingebaut werden, ohne die Keramik des WC-Beckens zu verändern. Der Stromanschluss kann sowohl aufputz als auch unterputz erfolgen.

Durch die Deodorisierungsvorrichtung werden nicht nur lästige Gerüche auf der Toilette vermieden, sondern findet auch eine Entkeimung der Luft statt.

Ausführungsbeispiele der Erfindung werden nachfolgend anhand der Zeichnungen näher erläutert. Es zeigen:
- Fig. 1: in einer schematischen Darstellung das Deodorisierungsmodul;
- Fig. 2: die Anordnung der einzelnen Bauelemente des Deodorisierungsmoduls auf einer Platine;
- Fig. 3: und 4 ein Ausführungsbeispiel der ersten Ausgestaltung der Erfindung;
- Fig. 5: bis 9 verschiedene Ausführungsbeispiele der zweiten Ausgestaltung der Erfindung;
- Fig. 10: ein Ausführungsbeispiel der dritten Ausgestaltung der Erfindung;
- Fig. 11: ein Ausführungsbeispiel der vierten Ausgestaltung der Erfindung;
- Fig. 12: und 13 ein Ausführungsbeispiel der fünften Ausgestaltung der Erfindung;
- Fig. 14: bis 16 das elektromagnetische Ventil zum Verhindern des Vordringens von Wasser zum Deodorisierungsmodul;
- Fig. 17: im Schnitt das Venturi-Ventil am Anfang der Abzweigung des Ansaugrohres;
- Fig. 18: das Kugel-Ventil am Anfang des Ansaugrohres;
- Fig. 19: und 20 das Kipp-Ventil am Anfang des Ansaugrohres und
- Fig. 21: das Torsions-Ventil am Anfang des Absaugrohres.

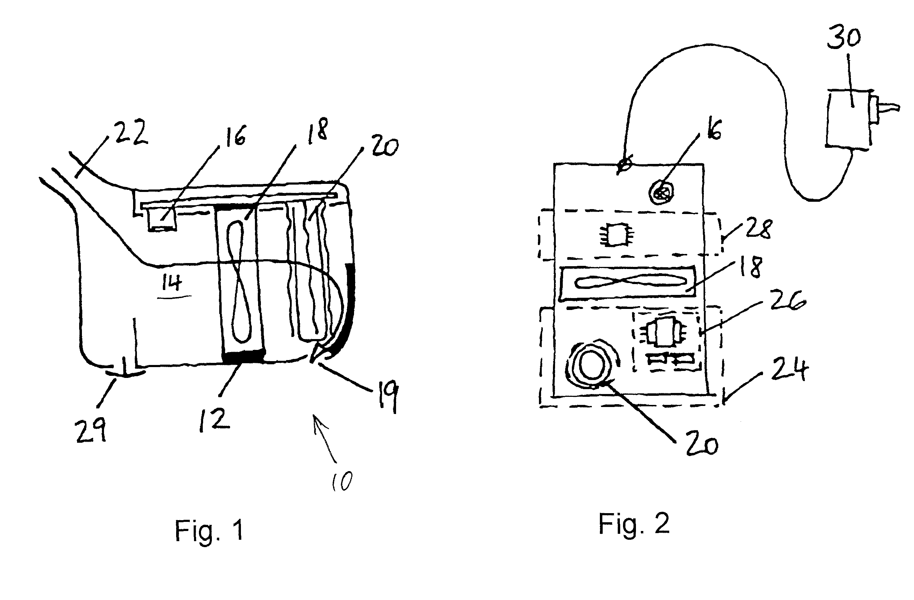

Wie in Fig. 1 gezeigt, ist das Deodorisierungsmodul (DOM) 10 in einem kompakten Gehäuse 12 untergebracht, durch das ein Strömungskanal 14 verläuft, in dem in Strömungsrichtung hintereinander ein Sensor 16, ein Lüfter 18 und eine Ionisationsröhre 20 angeordnet sind. Der Lüfter 18 kann auch an jeder anderen Stelle innerhalb des Strömungskanals 14 angeordnet sein. Er saugt Luft über ein Ansaugrohr 22 an, treibt sie durch den Strömungskanal 14 und bläst sie nach der Behandlung über eine Auslassöffnung 19 wieder in die Umgebung aus. Die Ionisationsröhre 20 ist Teil einer Ionisationseinrichtung 24 (Fig. 2), die noch eine Hochspannungseinheit 26 aufweist. Eine Steuereinheit 28 verarbeitet die Signale des Sensors 16 und steuert die Ionisationseinrichtung 24 in Abhängigkeit von den Sensorsignalen. Die Stromversorgung aller Komponenten erfolgt über ein Netzteil 30 in einem Netzstecker mit einer Ausgangsspannung von 12 Volt. Die Reihenfolge von Sensor 16, Lüfter 18 und Ionisationsröhre 20 ist dabei beliebig. Der Sensor 16, die Steuereinheit 28, der Lüfter 18, die Ionisationsröhre 20 und die Hochspannungseinheit 26 sind alle auf der gleichen Seite einer gemeinsamen Platine 32 montiert. Die Platine 32 ist oben in dem Gehäuse 12 befestigt, wobei die Montageseite mit den einzelnen Komponenten nach unten zeigt. Der Lüfter 18 füllt dabei den gesamten Querschnitt des Gehäuses 12 aus und auch die Ionisationsröhre 20 erstreckt sich bis nahe zur Unterseite des Gehäuses 12 (Fig. 1). Das Ansaugrohr 22 mündet auf der in Fig. 1 linken oberen Kante in das Gehäuse 12 und die Auslassöffnung 19 befindet sich an der rechten unteren Kante. Das DOM 10 hat an der Unterseite ein Notventil 29, über das eventuell eintretendes Wasser abgelassen wird. Das Notventil 29 ist als Pilzventil ausgeführt und öffnet bereits bei geringem Wasserdruck. Wie nachfolgend noch erläutert wird, sind die Mittel zum Ansaugen der Luft aus dem Beckeninneren jedoch so ausgelegt, dass kein Wasser in das DOM 10 eintreten kann.

Der Sensor 16 ist typischerweise ein Halbleiter-Zinnoxid-Sensor, der oxidierbare Gasbestandteile in der Luft feststellt. Der elektrische Widerstand des Sensors 16 ändert sich in Abhängigkeit von der Konzentration der oxidierbaren Luftbestandteile, die sich in der Luft befinden, die an den frei liegenden Oberflächen des Sensors 16 vorbei strömt.

Die Ionisationsröhre 20 besteht aus einem Glas- oder Keramikzylinder mit zwei Elektroden auf der Innenseite und der Außenseite. Die Hochspannungseinheit 26 erzeugt eine elektrische Wechselspannung von 1,5 kV und 10 kHz und diese Wechselspannung wird an die beiden Elektroden angelegt, wodurch Luftionen und aktiver Sauerstoff nach dem Prinzip der stillen oder dielektrisch behinderten Entladung erzeugt werden. Die Ionisationseinrichtung arbeitet im Impulsbetrieb, d.h. die Hochspannungseinheit 26 erzeugt eine die Hochspannung in Form einer Folge von Rechteck-Hochspannungsimpulsen. Dadurch wird erreicht, dass die Ionisationseinrichtung 24 immer mit der optimalen Spannung betrieben wird, bei der stabil und reproduzierbar überwiegend Luftionen und nur ein geringer Anteil an aktiven Sauerstoffatomen erzeugt werden.

Die Steuereinheit 28 steuert das DOM 10 in der Weise, dass im Ruhezustand, d.h., wenn der Sensor 16 kein Signal abgibt, das auf das Vorhandensein von oxidierbaren Luftbestandteilen hinweist, der Lüfter 18 etwa mit seiner halben Nennleistung läuft und die Ionisationseinrichtung 24 stromlos ist. Sobald der Sensor 16 meldet, dass oxidierbare Luftbestandteile in der angesaugten Luft vorhanden sind, wird der Lüfter 18 auf volle Leistung geschaltet und wird die Ionisationseinrichtung 24 eingeschaltet, wobei die Leistung der Ionisationseinrichtung 24 in Abhängigkeit von der Menge der oxidierbaren Luftbestandteile gesteuert wird. Die Steuereinheit 28 steuert die Leistung der Ionisationseinrichtung 24 durch Verändern der Impulsdauer und/oder der Impulsabstände, wobei die Maximal-Spannung jedes Impulses im Wesentlichen konstant bei etwa 1,5 kV liegt. Die Steuerung der Ionisationsleistung erfolgt in Abhängigkeit von dem Widerstandswert des Sensors 16 mit dem Ziel, gerade die Menge an Luftionen und Sauerstoffatome zu erzeugen, die notwendig ist, um die oxidierbaren Luftbestandteile zu oxidieren und damit zu zerstören. Die Sauerstoffatome führen zur Bildung von Ozon. Überschüssige Sauerstoffatome würden als beißender Ozongeruch wahrgenommen.

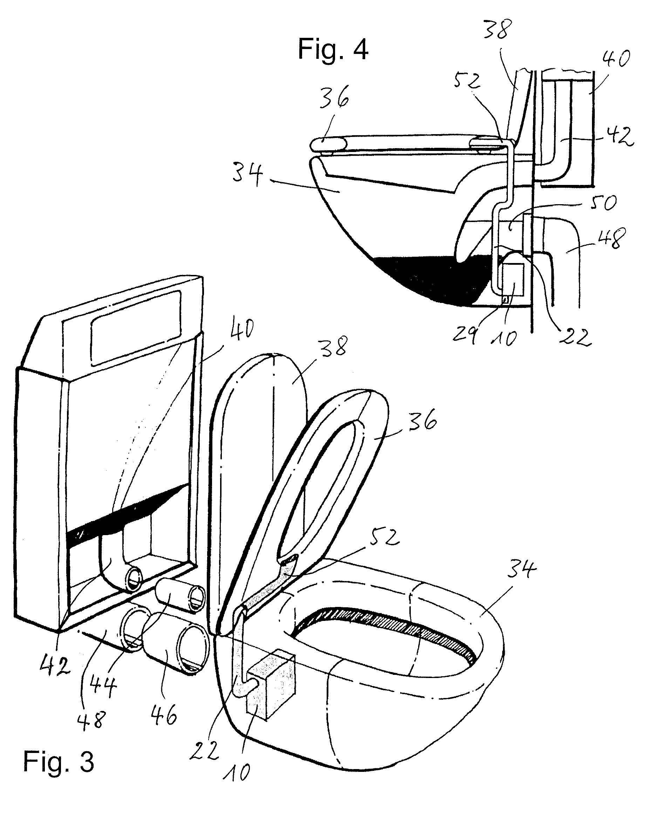

Die Figuren 3 bis 15 zeigen jeweils eine Toilettenausstattung mit einem WC-Becken 34, an dem ein Sitzbrett 36 und ein Deckel 38 in üblicher Weise mittels eines Scharniers befestigt sind. Die Toilette weist ferner einen Spülkasten 40 mit einem Spülrohr 42 auf, das in mehreren Auslassöffnungen am Rand des WC-Beckens 34 endet. Das Spühlrohr 42 kann zweiteilig sein und ein senkrechtes Fallrohr und ein waagerechtes Verbindungsstück 44 aufweisen. Die Entleerung des WC-Beckens 34 erfolgt über ein Abflussrohr 48, das an einem Abflussstutzen 50 des WC-Beckens 34 angesetzt ist. Ein Abflussverbindungsstück 46 kann dazwischen gesetzt sein. Der Spülkasten 40 weist die üblichen Bedienungsarmaturen auf, die in den Figuren jedoch nicht dargestellt sind. In allen Ausführungsbeispielen kommt dabei dasselbe DOM 10 zum Einsatz, wobei jeweils nur das Ansaugrohr 22 den unterschiedlichen Verhältnissen angepasst wird. Das Gehäuse 12 des DOM 10 ist insgesamt ein im Wesentlichen rechtwinkliger Körper mit einer maximalen Seitenlänge von etwa 100 mm.

Bei der in Fig. 3 und 4 gezeigten ersten Ausgestaltung der Erfindung wird die Luft über einen im Sitzbrett 36 verlaufenden Kanal 52 angesaugt. Die Einlassöffnung des Kanals 52 befindet sich am hinteren Ende der Öffnung des Sitzbrettes 36. Der Kanal 52 kann sich auch in eine Vielzahl von kleineren Kanälen auffächern, deren Einlassöffnungen über den Rand der Öffnung des Sitzbrettes 36 verteilt sind. Der Kanal 52 mündet über ein Drehrohrgelenk in das Ansaugrohr 22, das zum DOM 10 führt, das in einem Hohlraum im hinteren Bereich des WC-Beckens 34 angeordnet ist. Handelsübliche WC-Becken haben im hinteren Bereich über oder unter dem Abflussstutzen 50 einen geeigneten Hohlraum für die Unterbringung des DOM 10. Bei dieser Ausgestaltung der Erfindung erfolgt der Anschluss des WC-Beckens 34 an den Spülkasten 40 und das Abflussrohr 48 mittels üblicher Verbindungsteile und Manschetten.

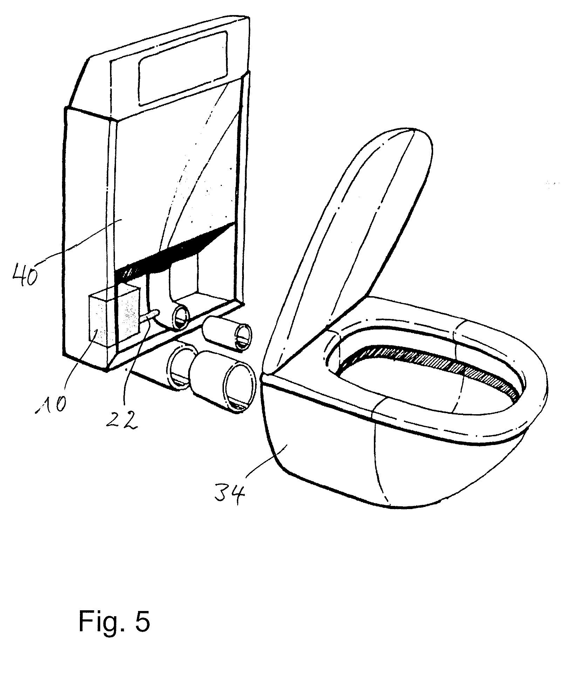

Bei dem in Fig. 5 dargestellten Ausführungsbeispiel der zweiten Ausgestaltung der Erfindung ist das Ansaugrohr 22 an der Stelle des Spülrohrs 42 angesetzt, an der dieses aus der Waagerechten nach oben abbiegt. Das Ansaugrohr 22 kann dabei stumpf mit dem Spülrohr 42 verbunden sein oder kann sich innerhalb des Spülrohrs 42 ganz oder teilweise bis zum WC-Becken 34 erstrecken. Das DOM 10 befindet sich unten im Spülkasten 40.

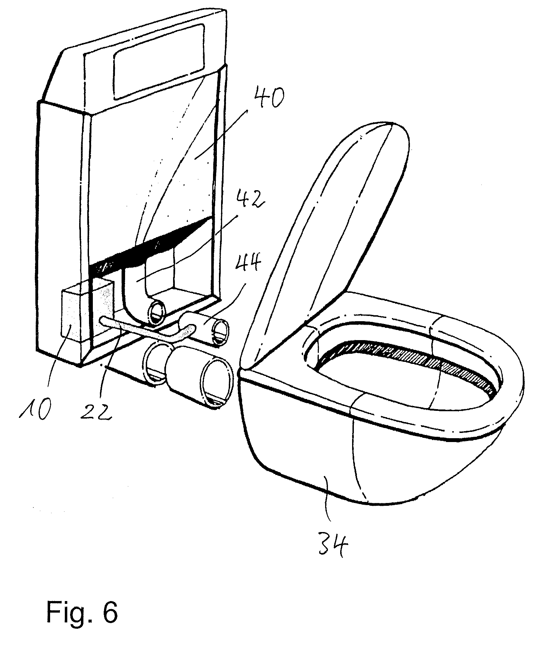

Bei der in Fig. 6 gezeigten Variante der zweiten Ausgestaltung ist der Anschluss des Ansaugrohrs 22 in Richtung WC-Becken 34 versetzt, und zwar mündet das Ansaugrohr 22 in das Verbindungsstück 44 des Spülrohrs 42 zum WC-Becken 34.

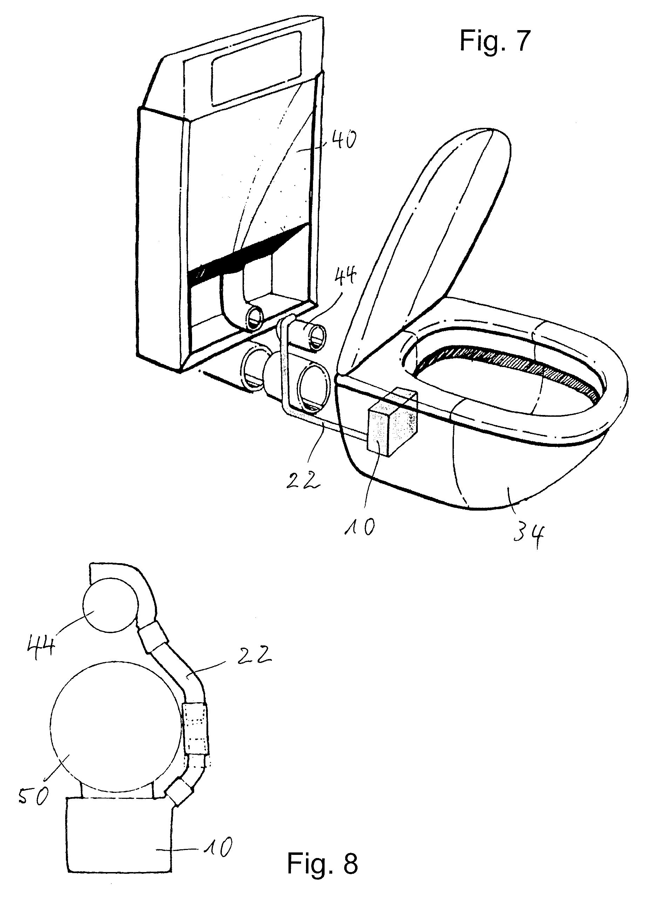

Bei der in Fig. 7 und 8 gezeigten Variante ist das Ansaugrohr 22 ebenfalls an das Verbindungsstück 44 angesetzt. Das DOM 10 wird mittels einer Manschette am Abflussstutzen 50 des WC-Beckens 34 abgehängt. Das Ansaugrohr 22 führt von der Oberseite des Verbindungsstücks 44 am Abflussstutzen 50 vorbei und mündet schräg von oben in das DOM 10 (Fig. 8). Ein Vorteil dieser Variante besteht darin, dass am Spülkasten 40 und der Keramik des WC-Beckens 34 keinerlei Änderungen erforderlich sind.

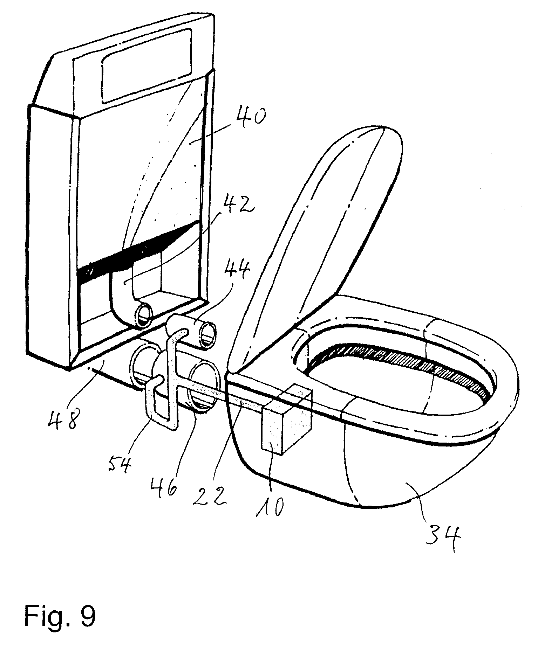

Bei der in Fig. 9 gezeigten Variante verbindet ein Verbindungsrohr 54 das Verbindungsstück 44 des Spülrohrs 42 mit dem Abflussrohr 48 oder dem Abflussverbindungsstück 46. Das Verbindungsrohr 54 ist dabei zunächst nach unten und dann wieder nach oben geführt, so dass ein Geruchsverschluss gebildet wird. Von diesem Verbindungsrohr 54 zweigt oberhalb des Niveaus des Abflussrohrs 48 das Ansaugrohr 22 ab und führt zum DOM 10. Durch das Verbindungsrohr 54 wird Wasser, das aus dem Spülrohr 42 oder dem Verbindungsstück 44 in das Verbindungsrohr 54 austritt, direkt in das Abflussrohr 48 abgeleitet, so dass es nicht den DOM 10 erreichen kann.

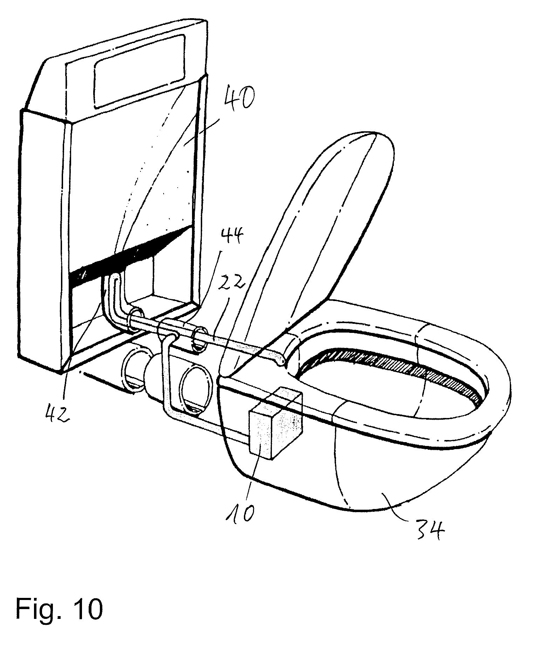

Eine andere Möglichkeit, das DOM 10 vor Wasser zu schützen, ist bei der dritten Ausgestaltung der Erfindung realisiert, die in Fig. 10 gezeigt ist. Das Einlassende des Ansaugrohrs 22 befindet sich am Rand des WC-Beckens 34. Das Ansaugrohr 22 verläuft von dort innerhalb des waagerechten Verbindungsstücks 44 und von dort ein Stück in den senkrechten Abschnitt des Spülrohrs 42 , kehrt dann nach unten um, tritt aus dem Verbindungsstück 44 seitlich aus und erreicht schließlich das DOM 10, das hierbei wiederum im hinteren Bereich des WC-Beckens 34 eingebaut ist. Der höchste Punkt des Ansaugrohrs 22, das ist die Umkehrschleife im senkrechten Abschnitt des Spülrohrs 42, liegt höher als der Rand des WC-Beckens 34, damit auch bei einem Überlauf des WC-Beckens 34 kein Wasser über das Ansaugrohr 22 zu dem DOM 10 fließt.

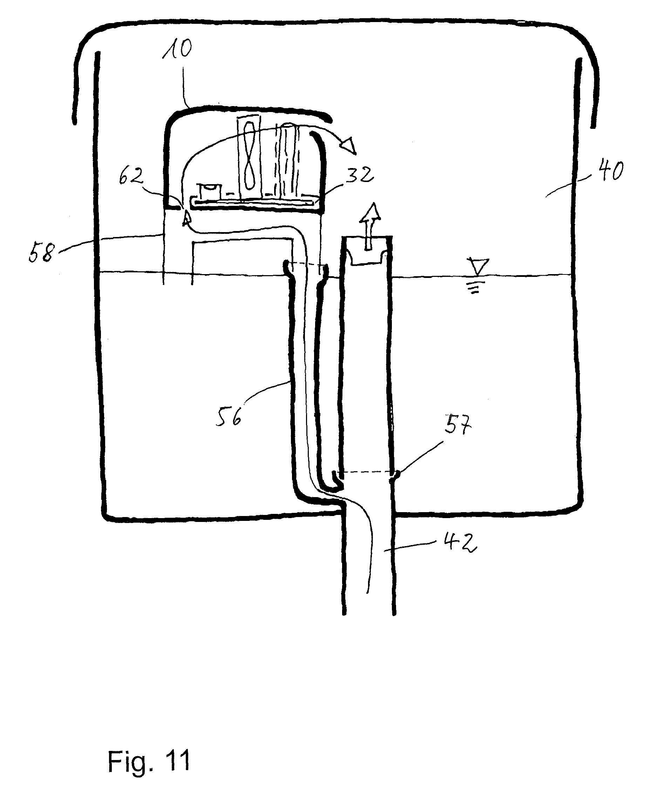

Bei der vierten Ausgestaltung der Erfindung, die in Fig. 11 gezeigt ist, ist das DOM 10 im oberen Bereich des Spülkastens 40 eingebaut. Bei dieser Bauart des Spülkastens ist ein Überlaufrohr 56 vorgesehen, das unterhalb des Spülventils 57 von dem Spülrohr 42 abzweigt und nach oben geführt ist. Zur Anordnung des DOM 10 ist auf das obere Ende des Überlaufrohrs 56 ein Adapter 58 aufgesetzt, in dem ein U-förmiger Kanal verläuft, so dass das obere Ende des Überlaufrohrs 56 nach unten umgeleitet wird. Durch den Adapter 58 behält das Überlaufrohr 56 seine Funktion bei. Bei einem drohenden Überlauf steigt das Wasser zunächst in dem freien Schenkel des U-förmigen Kanals hoch und fließt dann über das Überlaufrohr 56 ab. Der freie Schenkel ist soweit nach unten geführt, dass seine Öffnung bei normaler Füllhöhe des Spülkastens 40 in das Wasser eintaucht. Auf der Oberseite des Adapters 58 ist eine Öffnung 62 vorgesehen und das DOM 10 wird so auf den Adapter 58 aufgesetzt, dass durch die Öffnung 62 die Luft angesaugt wird. Aus dem Inneren des WC-Beckens 34 wird die Luft somit über das Spülrohr 42, das Überlaufrohr 56 und den damit verbundenen Schenkel des U-förmigen Kanals des Adapters 58 und durch die Öffnung 62 hindurch angesaugt. Während der Toilettenspülung kann keine Luft aus dem Inneren des WC-Beckens 34 angesaugt werden. Wenn überschüssiges Wasser über den Adapter 58, das Überlaufrohr 56 und das Spülrohr 42 abfließt, hängt es von der Menge des abfließenden Wassers ab, ob noch ein freier Querschnitt innerhalb dieser Rohre für das in Gegenrichtung erfolgende Absaugen von Luft aus dem Inneren des WC-Beckens 34 vorhanden ist. Das DOM 10 wird bei dieser Ausführungsform im Allgemeinen so auf den Adapter 58 aufgesetzt, dass sich die Platine 32 im unteren Bereich des Gehäuses 10 befindet.

Sobald der Wasserspiegel beim Spülen soweit abgesunken ist, dass der freie Schenkel des U-förmigen Kanals des Adapters 58 nicht mehr ins Wasser eintaucht, so wird durch diesen Schenkel Luft angesaugt, die dann jedoch nicht aus dem Inneren des WC-Beckens 34 kommt.

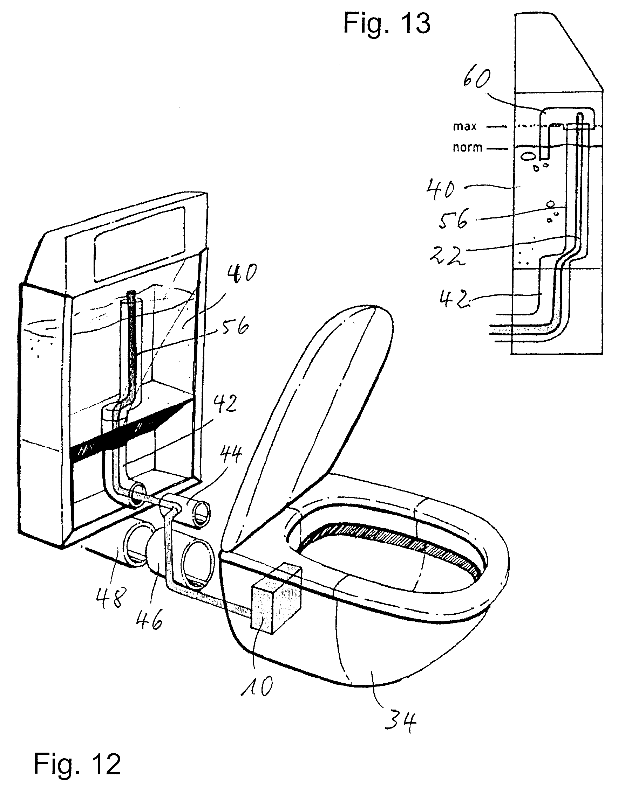

Eine fünfte Ausgestaltung der Erfindung ist in den Figuren 12 und 13 gezeigt. Der Spülkasten 40 mit dem Spülrohr 42 und dem Überlaufrohr 56 ist so wie bei der vierten Ausgestaltung der Erfindung ausgebildet. Das Ansaugrohr 22 verläuft jedoch innerhalb des Überlaufrohrs 56, und dann weiter im Spülrohr 42, tritt aus dessen Verbindungsstück 44 seitlich aus und führt zum DOM 10, das wiederum mittels einer Manschette vom Abflussrohr 48 abgehängt ist. Das Einlassende des Ansaugrohrs 22 steht nach oben aus dem Überlaufrohr 56 vor. Um zu verhindern, dass das Ansaugrohr 22 Luft aus dem Spülkasten 40 ansaugt, ist auf das Überlaufrohr 56 ein U-Stück 60 aufgesetzt, das ähnlich dem Adapter 58 der vierten Ausgestaltung der Erfindung ausgebildet ist, jedoch keine Öffnung 62 an der Oberseite aufweist. Bei normaler Füllung des Spülkastens 40 ist das obere Ende des Überlaufrohrs 56 für Lufteintritt aus dem Spülkasten 40 verschlossen, so dass das Ansaugrohr 22 bei vollem Spülkasten Luft nur über das Überlaufrohr 56 und das Spülrohr 42 aus dem Inneren des WC-Beckens 34 ansaugen kann.

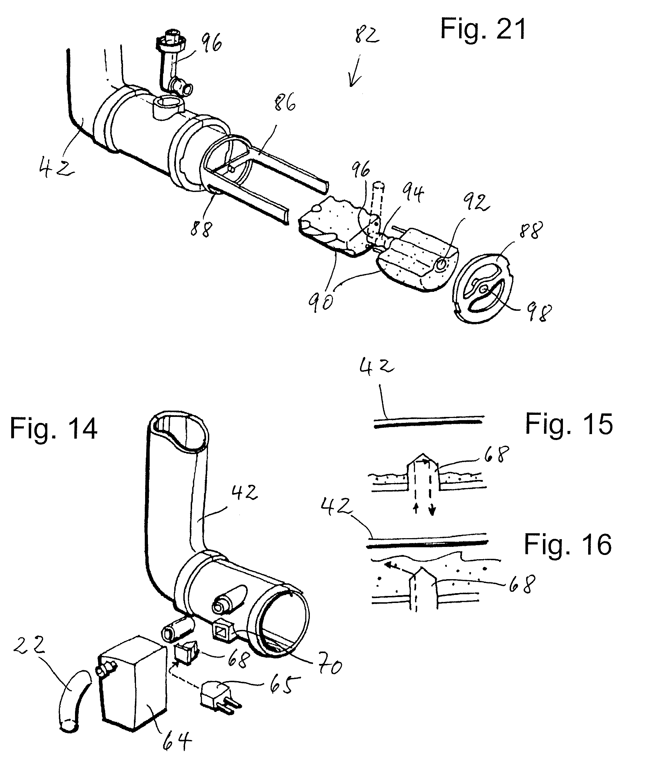

Die Figuren 14 bis 16 zeigen eine elektromagnetische Ventileinrichtung und deren Funktionsweise, wobei das elektromagnetische Ventil an der Verbindungsstelle zwischen Spülrohr 42 und Ansaugrohr 22 eingesetzt wird, um die Verbindung zwischen beiden Rohren beim Spülen der Toilette zu schließen, so dass kein Wasser aus dem Spülrohr 42 in das Ansaugrohr 22 eintreten kann. Die elektromagnetische Ventileinrichtung weist ein Ventil 64 auf sowie einen Wassersensor 65. Das Ventil 64 ist von der üblichen Bauart eines elektromagnetischen Ventils und enthält ein Solenoid. Der Wassersensor 65 weist einen Strahler und Empfänger auf, die in einer Einheit kombiniert sind. Die Einheit ist über ein zweiadriges Lichtleiterkabel mit einem Prisma-Element 68 verbunden. Wenn sich das Prisma-Element 68 in Luft befindet (Fig. 15), so wird der von dem Strahler über das Kabel zugeführte Lichtstrahl totalreflektiert und zum Empfänger zurückgeschickt. Befindet sich das Prisma-Element 68 jedoch in Wasser (Fig. 16), so tritt keine Totalreflexion auf, so dass der Empfänger kein Licht erhält. Das Prisma-Element 68 wird über eine Durchführung 70 in das Spülrohr 42 eingesetzt. Über eine Steuereinheit wird das Signal des Empfängers so umgesetzt, dass das Ventil 64 nur dann öffnet, wenn sich im Spülrohr 42 kein Wasser befindet. Diese elektromagnetische Ventileinrichtung kann bei der zweiten und dritten Ausgestaltung der Erfindung gemäß den Figuren 2 bis 8 eingesetzt werden.

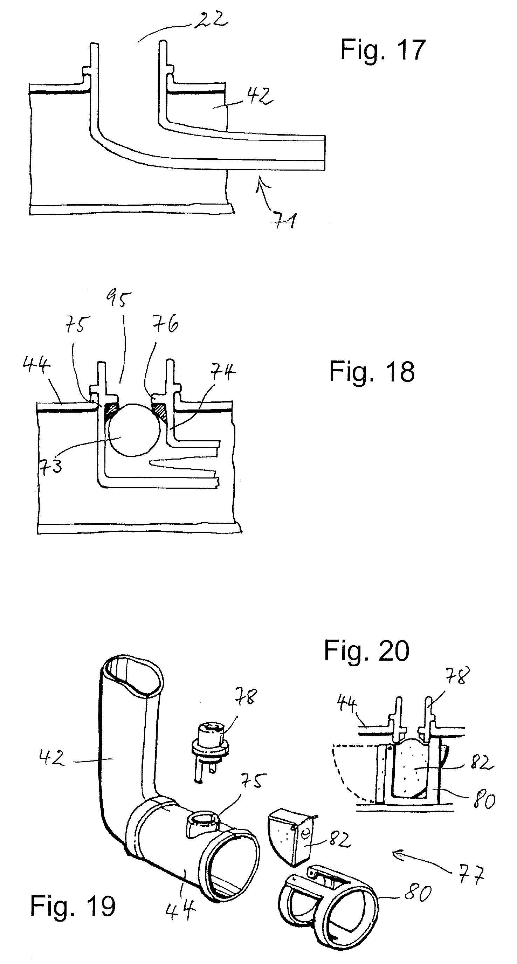

Figur 17 zeigt ein Venturi-Ventil 71, das eine mechanisch besonders einfache Lösung dafür darstellt, den Eintritt von Wasser in das Ansaugrohr 22 zu verhindern. Das Einlassende des Ansaugrohrs 22 ist dazu im Spülrohr 42 ein Stück in Strömungsrichtung des Spülwassers geführt. Beim Auslösen des Spülvorgangs entsteht dadurch im Ansaugrohr 22 ein Unterdruck, durch den eventuell vorhandenes Wasser oder Kondenswasser abgesaugt wird.

Fig. 18 zeigt ein Kugel-Ventil, dass denselben Zweck erfüllt. Eine Kugel 73, die ein geringeres spezifisches Gewicht als Wasser hat, bewegt sich dabei innerhalb einer Fassung 74 frei. Das Kugelventil 72 ist auf der Oberseite des Verbindungsstücks 44 in einen Stutzen 75 eingesetzt. Wenn sich im Spülrohr 42 Wasser befindet, so schwimmt die Kugel 73 auf dem Wasser und drückt dadurch dichtend nach oben gegen eine Schulter 76 innerhalb der Fassung 74. Oberhalb der Schulter 76 befindet sich eine Kompensationskammer 95. Die Kompensationskammer 95 kann eine geringe Menge Wasser aufnehmen, die beim Spülen ggf. durch das Kugel-Ventil hindurch tritt. Ähnlich wie bei dem Venturi-Ventil 71 ist der sich innerhalb des Spülrohrs 42 befindende Teil der Fassung 74 in Strömungsrichtung des Spülwassers umgebogen, wobei allerdings bei dem Kugelventil 72 dieser Teil seitlich geschlitzt ist, damit die Kugel 73 mit dem Wasser in Berührung kommt.

Fig. 19 und 20 zeigen ein Kippventil 77. Das Kippventil 77 weist eine Ventilaufnahme 78 auf, die in den Anschlussstutzen 75 am Verbindungsstück 44 eingesetzt wird. Die Ventilaufnahme 78 endet unten in einer ringförmigen Dichtungsfläche. In das Verbindungsstück 44 wird ein Führungskäfig 80 eingeschoben, in dem ein torten- oder käsestückähnlicher Schwimmer 82 pendelnd aufgehängt ist, wobei die Pendelachse an der Spitze liegt, d.h. die Mittelachse der gedachten Torte ist. Der Schwimmer 82 ist im Inneren inhomogen aufgebaut, so dass sein Schwerpunkt nahe der Schnittfläche liegt, die von der Spülströmung abgewandt ist. Im Ruhezustand, d.h., wenn sich kein Wasser im Spülrohr 42 befindet, nimmt der Schwimmer daher die in Fig. 20 gestrichelt eingezeichnete Position ein, wobei die Öffnung der Ventilaufnahme 78 frei liegt. Wenn Spülwasser durch das Spülrohr 42 und das Verbindungsstück 44 strömt, so drückt das Wasser den Schwimmer 82 in die Position, die in Fig. 20 durchgezogen gezeichnet ist. In dieser Position verschließt der Schwimmer 82 die Öffnung der Ventilaufnahme 78. Damit der Schwimmer 82 sicher an der Öffnung der Ventilaufnahme anliegt, kann er an der Stelle, die im geschlossenen Zustand innerhalb der Öffnung der Ventilaufnahme 78 liegt, eine ballige Erhebung aufweisen.

Figur 21 zeigt ein Torsionsventil 84. Das Torsionsventil weist einen rechteckigen Rahmen 86 auf, der in das Spülrohr 42 eingesetzt wird und am vorderen und hinteren Ende ringförmige Abstützungen 88 aufweist. Ein zweiteiliger Schwimmer 90 ist in dem Rahmen 86 drehbar gelagert, wobei die Drehachse mit der Längsachse des Spülrohrs 42 zusammenfällt. Die beiden Teile des Schwimmers sind Halbzylinder und in dem hinsichtlich der Spülströmung stromabwärts liegenden Halbzylinder ist ein zur Drehachse koaxialer Kanal 92 vorgesehen. An dem zwischen den beiden Teilen des Schwimmers liegenden Ende des Kanals 92 ist ein flexibles Gummirohr 94 angeschlossen, das mit einem Winkelstück 96 verbunden ist, das wiederum nach oben aus dem Spülrohr 42 herausgeführt ist und mit dem Ansaugrohr 22 verbunden wird. Die beiden Teile des Schwimmers 90 werden soweit zusammengesteckt, dass das Gummirohr 94 zwischen ihnen noch herausgeführt werden kann. Am stromabseitigen Ende ist der Schwimmer 90 so gelagert, dass eine axiale Öffnung 98 frei bleibt. Durch diese Öffnung 98, den Kanal 92, das Gummirohr 94 und das Winkelstück 96 besteht damit ein freier Kanal, der sich im Ansaugrohr 22 fortsetzt. Wenn sich im Spülrohr 42 kein Wasser befindet, so dreht sich der Schwimmer 90 durch sein Gewicht nach unten. Das Gummirohr 94 ist so eingesetzt, dass es dann faltenfrei ist. Beim Spülen dreht sich der Schwimmer 90 durch seinen Auftrieb um 180° nach oben. Das Gummirohr 94 wird dadurch verdrillt, so dass es den Durchgang blockiert und dann keine Luft mehr durch das Ansaugrohr 22 angesaugt werden kann. Der stromaufwärtige Teil des Schwimmers 90 hat wendelförmige Rillen oder Stege auf seiner halbzylindrischen Oberfläche, wodurch die Drehrichtung festgelegt wird, in der sich der Schwimmer 90 um seine Längsachse nach oben dreht. Die Steckverbindung zwischen den beiden Teilen des Schwimmers 90 ist so ausgebildet, dass diese Drehung nicht behindert wird, d.h. die Steckverbindungselemente sind auf den Viertelkreis beschränkt, der sich bei der Drehung zunächst nach unten dreht.

Statt des Gummirohrs 94 können auch zwei halbkreisförmige Blenden gegeneinander verdreht werden. Bspw. kann die Öffnung 98 halbkreisförmig sein und der Kanal 92 oder zumindest sein Anfang ebenfalls halbkreisförmig sein. Die halbkreisförmigen Öffnungen sind dabei so angeordnet, dass sie fluchten, wenn sich der Schwimmer 90 in seiner unteren Lage befindet, so dass eine freie Verbindung besteht. Befindet sich der Schwimmer in seiner oberen Lage, so sind die halbkreisförmigen Öffnungen um 180° gegeneinander verdreht, so dass die Verbindung blockiert ist.

### Bezugszeichenliste

- 10: DOM
- 12: Gehäuse
- 14: Strömungskanal
- 16: Sensor
- 18: Lüfter
- 19: Auslassöffnung
- 20: Ionisationsröhre
- 22: Ansaugrohr
- 24: Ionisierungseinrichtung
- 26: Hochspannungseinheit
- 28: Steuereinheit
- 29: Notventil
- 30: Netzteil
- 32: Platine
- 34: WC-Becken
- 36: Sitzbrett
- 38: Deckel
- 40: Spülkasten
- 42: Spülrohr
- 44: Verbindungsstück
- 46: Abflussverbindungsstück
- 48: Abflussrohr
- 50: Abflussstutzen
- 52: Kanal
- 54: Verbindungsrohr
- 57: Spülventil
- 56: Überlaufrohr
- 58: Adapter
- 60: U-Stück
- 62: Öffnung
- 64: Ventil
- 65: Wassersensor
- 68: Prisma-Element
- 70: Durchführung
- 71: Venturi-Ventil
- 72: Kugelventil
- 73: Kugel
- 74: Fassung
- 75: Stutzen
- 76: Schulter
- 77: Kippventil
- 78: Ventilaufnahme
- 80: Führungskäfig
- 82: Schwimmer
- 84: Torsionsventil
- 86: Rahmen
- 88: Abstützung
- 90: Schwimmer
- 92: Kanal
- 94: Gummirohr
- 95: Kompensationskammer
- 96: Winkelstück
- 98: Öffnung

## Patentansprüche

1. Toilettenausstattung mit einem WC-Becken (34) und einer Spüleinrichtung (40), die über ein Spülrohr (42) das WC-Becken (34) spült, und mit einer Deodorisierungseinrichtung (10), die mittels eines Lüfters (18) über ein Ansaugrohr (22) Luft aus dem Inneren des WC-Beckens (34) absaugt, wobei die Deodorisierungseinrichtung eine Ionisationseinrichtung (24) zum Erzeugen von Luftionen aufweist und die abgesaugte Luft mit den erzeugten Luftionen in Kontakt gebracht wird, **gekennzeichnet durch** einen Sensor (16), der die abgesaugte Luft auf oxidierbare Geruchsmoleküle untersucht und ein Signal erzeugt, das die Konzentration oxidierbarer Geruchsmoleküle in der abgesaugten Luft wiedergibt, wobei die Ionisationseinrichtung (24) in Abhängigkeit von dem Signal des Sensors (16) gesteuert wird.

2. Toilettenausstattung nach Anspruch 1, wobei die Steuerung der Ionisationseinrichtung (24) beinhaltet, dass die Ionisationseinrichtung (24) aktiviert wird, sobald der Sensor (16) anzeigt, dass in der abgesaugten Luft oxidierbare Geruchsmoleküle enthalten sind.

3. Toilettenausstattung nach Anspruch 1 oder 2, wobei der Lüfter (18) so gesteuert wird, dass er mit einer ersten Leistung arbeitet, wenn das Signal des Sensors (16) keine oxidierbaren Geruchsmoleküle in der abgesaugten Luft anzeigt, und dass er mit einer zweiten, höheren Leistung arbeitet, wenn das Signal das Vorhandensein oxidierbarer Geruchsmoleküle in der abgesaugten Luft anzeigt.

4. Toilettenausstattung nach einem der Ansprüche 1 bis 3, wobei ein Aktivkohlefilter vorgesehen ist, durch das der Luftstrom nach dem Durchgang durch die Ionisationseinrichtung (24) hindurch geleitet wird.

5. Toilettenausstattung nach einem der Ansprüche 1 bis 4, wobei das Ansaugrohr (22) von dem Spülrohr (42) abzweigt und die Deodorisierungseinrichtung (10) im hinteren Bereich des WC-Beckens oder in einem gegebenenfalls vorhandenen Spülkasten (40) angeordnet ist.

6. Toilettenausstattung nach einem der Ansprüche 1 bis 4, wobei das Spülrohr (42) über ein Verbindungsrohr (54) mit dem Abfluss (48) des WC-Beckens (34) verbunden ist und das Ansaugrohr (22) von dem Verbindungsrohr (54) abzweigt.

7. Toilettenausstattung nach einem der Ansprüche 1 bis 4, wobei das Ansaugrohr (22) in das Spülrohr (42) eintritt und bis zum Rand des WC-Beckens (34) geführt ist.

8. Toilettenausstattung nach einem der Ansprüche 1 bis 4, mit einem Spülkasten (40), wobei die Deodorisierungseinrichtung (10) im Spülkasten (40) mittels eines Adapters (58) am oberen Ende des Überlaufrohrs (56) angeordnet ist, wobei der Adapter (65) bei gefülltem Spülkasten (40) verhindert, dass die Deodorisierungseinrichtung (10) Luft aus dem Spülkasten (40) ansaugt.

9. Toilettenausstattung nach einem der Ansprüche 1 bis 4, mit einem Spülkasten (40), wobei das Ansaugrohr (22) im Spülrohr (42) und einem gegebenenfalls vorhandenen Überlaufrohr (56) bis über das Flutungsniveau des Spülkastens (40) geführt ist.

10. Toilettenausstattung nach Anspruch 9, wobei das Einlassende des Spülrohrs (42) und das dort liegende Ende des Ansaugrohrs (22) so abgedeckt sind, dass bei vollem Spülkasten (40) das Ansaugrohr (22) über das Spülrohr (22) und das Überlaufrohr (56) Luft nur aus dem WC-Becken (34) ansaugen kann.

11. Toilettenausstattung nach einem der Ansprüche 1 bis 10, wobei das Ansaugrohr (22) so geführt ist, dass der Eintritt von Wasser in das Deodorisierungsmodul (10) verhindert wird oder elektrische oder mechanische Ventile (64, 72, 77, 84) oder eine Venturidüse (71) vorgesehen ist, die das Eindringen von Wasser in das Deodorisierungsmodul (10) verhindern.
